(19) Europäisches Patentamt
European Patent Office
Office européen des brevets

(11) EP 1 127 890 A1

(12) EUROPEAN PATENT APPLICATION

(43) Date of publication:
29.08.2001 Bulletin 2001/35

(51) Int Cl.[7]: C07J 71/00, A61K 31/58, A61P 15/08, A61K 31/585

(21) Application number: 00250059.3

(22) Date of filing: 22.02.2000

(84) Designated Contracting States:
AT BE CH CY DE DK ES FI FR GB GR IE IT LI LU MC NL PT SE
Designated Extension States:
AL LT LV MK RO SI

(71) Applicant: SCHERING AKTIENGESELLSCHAFT
13353 Berlin (DE)

(72) Inventors:
- Esperling, Peter
  12107 Berlin (DE)
- Kuhnke, Joachim
  14469 Potsdam (DE)
- Blume, Thorsten
  13467 Berlin (DE)
- Hegele-Hartung, Christa
  45470 Mühlheim/Ruhr (DE)

(54) **Steroid derivatives with an additional ring annulated to ring A and use of these derivatives for the preparation of meiosis regulating medicaments**

(57) The present invention relates to pharmaceutically active steroid derivatives with an additional ring annulated to ring A, to pharmaceutical compositions comprising and to the use of these compounds for the preparation of medicaments. More particularly it has been found that the described steroids of the invention can be used for regulating meiosis.

EP 1 127 890 A1

## Description

The present invention relates to pharmaceutically active steroid derivatives, to pharmaceutical compositions comprising them as active substances and to the use of these compounds for the preparation of medicaments. More particularly it has been found that the steroid derivatives of the invention can be used for regulating meiosis.

Meiosis is the unique and ultimate event of germ cells on which sexual reproduction is based. Meiosis comprises two meiotic divisions. During the first division, exchange between maternal and paternal genes takes place before the pairs of chromosomes are separated into the two daughter cells. These contain only half the number (1n) of chromosomes and 2c DNA. The second meiotic division proceeds without a DNA synthesis. This division therefore results in the formation of the haploid germ cells with only 1c DNA.

The meiotic events are similar in the male and female germ cells, but the time schedule and the differentiation processes which lead to ova and to spermatozoa differ profoundly. All female germ cells enter the prophase of the first meiotic division early in life, often before birth, but all are arrested as oocytes later in the prophase (dictyate state) until ovulation after puberty. Thus, from early life the female has a stock of oocytes which is drawn upon until the stock is exhausted. Meiosis in females is not completed until after fertilization, and results in only one ovum and two abortive polar bodies per germ cell. In contrast, only some of the male germ cells enter meiosis from puberty and leave a stem population of germ cells throughout life. Once initiated, meiosis in the male cell proceeds without significant delay and produces 4 spermatozoa.

Only little is known about the mechanism which controls the initiation of meiosis in the male and in the female. In the oocyte, new studies indicate that follicular purines, hypoxanthine or adenosine, could be responsible for meiotic arrest [Downs, S.M. *et al. Dev Biol* **82** (1985) 454-458: Epplg. J. J. et al *Dev Biol* **119** (1986) 313-321; and Downs, S. M. *Mol Reprod Dev* **35** (1993) 82-94]. The presence of a diffusible meiosis regulating substance was first described by Byskov *et al.* in a culture system of fetal mouse gonads [Byskov, A. G. *et al*. *Dev Biol* **52** (1976) 193-200]. A meiosis activating substance (MAS) was secreted by the fetal mouse ovary in which meiosis was ongoing, and a meiosis preventing substance (MPS) was released from the morphologically differentiated testis with resting, non-meiotic germ cells. It was suggested that the relative concentrations of MAS and MPS regulated the beginning, arrest and resumption of meiosis in the male and in the female germ cells (Byskov, A.G. et al. in *The Physiology of Reproduction* [eds. Knobil. E. and Neill, J. D., Raven Press, New York (1994)]. Clearly, if meiosis can be regulated, reproduction can be controlled. A recent article [Byskov, A. G. et al. *Nature* **374** (1995), 559-562] describes the isolation from bull testes and from human follicular fluid of certain sterols (T-MAS and FF-MAS) that activate oocyte meiosis. Unfortunately, these sterols are rather labile.

Compounds being known to regulate the meiosis are described in the International patent applications WO 96/00235, WO 96/27658, WO97/00884, WO98/28323,WO98/52965 and WO 98/55498.

It is purpose of the present invention to provide a new class of compounds useful for regulating meiosis in females and males, particularly in humans.

The present invention relates to steroids of the general formula I:

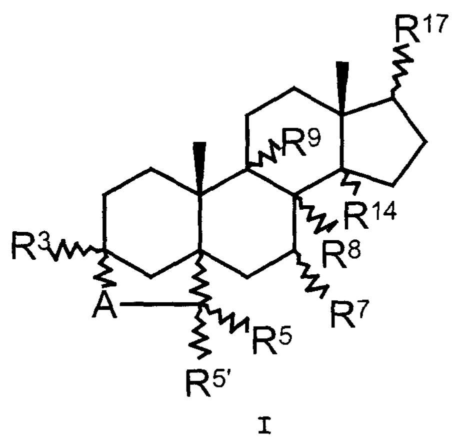

I

wherein

- $R^3$ designates a hydrogen atom, a hydroxy group or a 2-hydroxyethoxy group,
- A designates an oxygen atom or a NH group,
- $R^5$ designates a hydrogen atom, a hydroxy group, an $C_1$-$C_8$ linear or branched alkyl or alkenyl group, a $C_6$-$C_{10}$ aryl

group or together with $R^5$ an oxo group,

$R^{5'}$ designates a hydrogen atom or together with $R^5$ an oxo group,

$R^7$ designates a hydrogen atom or together with $R^8$ an additional bond,

$R^8$ designates a hydrogen atom or together with R7 or together with R9 or together with $R^{14}$ an additional bond,

$R^9$ designates a hydrogen atom or together with $R^8$ an additional bond,

$R^{14}$ designates a hydrogen atom or together with $R^8$ or together with $R^{15}$ an additional bond,

$R^{17}$ a $C_1$-$C_{10}$ linear or branched optionally substituted alkyl or alkenyl group, an optionally substituted $C_8$-$C_{12}$ arylalkyl group, a $C_1$-$C_{10}$ linear or branched optionally substituted alkoxy group or an optionally substituted $C_6$-$C_{10}$ arylalkoxy group

or isomeric forms, salts or esters thereof with the provisio that said compounds are not 5β-cholestane-5, 3β-carbolactone, 5α-cholestane-5,3α-carbolactone, 3α-hydroxy-5β-cholestane-5,3β-carbolactame, 3β-hydroxy-5α-cholestane-5, 3α-carbolactame or 3α-(2-hydroxyethoxy)-5β-cholestane-5,3β-carbolactame.

As used in the present description and claims, an alkyl group - when used alone or in combinations - may be a straight or branched alkyl group. The expression $C_1$-$C_8$ alkyl designates an alkyl group having from one to eight carbon atoms: preferred examples are methyl, ethyl, propyl, isopropyl, butyl, iso-butyl, tert-butyl, pentyl, iso-pentyl, hexyl and cyclohexyl. The term alkenyl group refers to an unsaturated alkyl group. Preferred examples are vinyl, allyl, isopropenyl and prenyl. The term $C_6$-$C_{10}$ aryl group designates a phenyl group, that is optionally substituted by halogen, ($C_1$-$C_4$)alkoxy, hydroxy or ($C_1$-$C_4$)alkyl groups. As used in the present description and claims, a statement that e.g. $R^5$ together with $R^{5'}$ is an oxo group means that oxo (=O) is present in the 5 position and, consequently, there is no hydrogen atom in the 5 position. The term halogen means fluoro, chloro, bromo or iodide. The expression $C_1$-$C_{10}$ alkyl designates an alkyl group having from one to ten carbon atoms: preferred examples are methyl, ethyl, propyl, isopropyl, butyl, iso-butyl, tert-butyl, pentyl, iso-pentyl, hexyl and cyclohexyl. Sterol side chains like cholesterol, Δ-24-cholesterol and ergosterol side chains are also preferred for $R^{17}$. A $C_8$-$C_{12}$ arylalkyl group stands for an optionally substituted aryl group that is connected via an alkyl spacer group to the steroidal nucleus. A $C_1$-$C_{10}$ alkoxy group designates an aliphatic ether group having one to ten carbon atoms. This group may be unsaturated. Preferred examples are methoxy, ethoxy, i-propoxy, allyloxy and prenyloxy. A $C_6$-$C_{10}$ arylalkoxy group designates an ether group with six to ten carbons having an aromatic ring in the side chain. Preferred examples are phenoxy, benzyloxy and phenethyloxy.

Salts of compounds of formula I and II are preferably pharmaceutically acceptable salts. Examples are listed in Journal of Pharmaceutical Science, (1977) 66, 2 et seq. Examples of such salts include salts of organic acids such as formic acid, fumaric acid, acetic acid, propionic acid, glycolic acid, lactic acid, pyruvic acid, oxalic acid, succinic acid, malic acid, tartaric acid, citric acid, benzoic acid, salicylic acid and the like. Suitable inorganic acid-addition salts include salts of hydrochloric, hydrobromic, sulphuric and phosphoric acids and the like.

Esters of compounds of formula I and II are formally derived by esterification of one or more hydroxylic groups of a compound of formula I or II with an acid which can for example be selected from the group of acids comprising succinic acid and other dicarboxylic acids, nicotinic acid, isonicotinic acid, ethylcarbonic acid, phosphoric acid, sulphonic acid, sulphamic acid, benzoic acid, acetic acid, propionic acid and other aliphatic monocarboxylic acids.
The compounds of the general formula I have a number of chiral centres in the molecule and thus exist in several isomeric forms. All these isomeric forms and mixtures thereof are within the scope of the invention (unless otherwise noted).

Preferred compounds of formula I are such wherein $R^3$ designates a hydrogen atom or a hydroxy group.

Other preferred compounds of formula I are such wherein $R^5$ and $R^{5'}$ designate together an oxo group.

Other preferred compounds of formula I are such wherein $R^{17}$ designates a $C_1$-$C_{10}$ linear or branched optionally substituted alkyl or alkenyl group or a $C_1$-$C_{10}$ linear or branched optionally substituted alkoxy group. Especially preferred compounds of formula I are such wherein $R^{17}$ designates a prenyloxy group.

The following new compounds of formula I are especially preferred :

5β-cholest-8-ene-5,3β-carbolactone
5β-cholest-8(14)-ene-5,3β-carbolactone
3α-hydroxy-5β-cholest-8-ene-5,3β-carbolactame
3α-hydroxy-5β-cholest-8(14)-ene-5,3β-carbolactame
2-[(4aα-methyl-4a-homo-3α,5-methano-4-oxa-5α-cholest-8-en-3β-yl)oxy]ethanol
2-[(4aα-ethyl-4a-homo-3a,5-methano-4-oxa-5α-cholest-8-en-3β-yl)oxy]ethanol
2-[(4aα-methyl-4a-homo-3α,5-methano-4-oxa-24-nor-5α-cholest-8-en-3β-yl)oxy]ethanol
2-[(20R)-(4aα,20-dimethyl-4a-homo-3α,5-methano-4-oxa-21-phenyl-5α-pregn-8-en-3β-yl)oxy]ethanol
2-[(20R)-(21-cyclohexyl-4aa,20-dimethyl-4a-homo-3α,5-methano-4-oxa-5α-pregn-8-en-3β-yl)oxy]ethanol
2-[(4aα-methyl-4a-homo-3α,5-methano-4-oxa-5α-cholest-8(14)-en-3β-yl)oxy]ethanol
24-nor-5β-cholestane-5,3β-carbolactone

(20R)-21-cyclohexyl-20-methyl-5β-pregnane-5,3β-carbolactone
17β-(prenyloxy)-5β-androstane-5,3β-carbolactone

It was surprisingly found that the compounds of this invention, which are structurally highly different from the endogenous meiosis activating sterol [FF-MAS, Byskov, A. G. et al. *Nature* **374** (1995), 559-562] have a strong meiosis regulating effect.

Some of the here claimed compounds show an extraordinary high proportion of polar body formation in the oocyte test. Polar body formation is an indication of meiosis activation. In this respect the compounds of this invention are superior to the formerly described agonistic compounds. Preferred in this context are compounds of formula I with $R^3$ designating a hydroxy group, A designating an NH group and with $R^{17}$ being a sterol side chain. Compounds with $R^{17}$ being the cholesterol side chain are still more preferred. Especially preferred as such an "polar body superagonist" is 3α-hydroxy-5β-cholest-8-en-5,3β-carbolactame.

On the other hand, most compounds of this invention strongly antagonize the effect of the naturally occuring FF-MAS and are thus inhibitors of meiosis. These findings make the described compounds especially interesting for contraception. Preferred compounds of formula I are those which inhibit the germinal vesicle breakdown by at least 20 %, preferably at least 40 %, especially preferred at least 60 % when tested in an oocyte test as described in example 13 and which do not activate meiosis in an oocyte test as described in example 12.

Some antagonistic compounds can also directly inhibit the meiotic maturation. This means they can not only inhibit the FF-MAS induced maturation of oocytes in an inhibitory hypoxanthine medium, but they also can play the role of the meiosis preventing substance hypoxanthine themselves. Whereas hypoxanthine has to be present in the maturation medium in milimolar concentrations to excert its inhibiting effect, the antagonistic compounds of the present invention have their direct antagonistic potential in the micromolar range. These findings have not been described before. Because of their dual inhibitory potential the described antagonists are superior to formerly described antagonists. Preferred in this context are compounds of formula I with $R^3$ designating a 2-hydroxyethoxy group, A designating an oxygen atom, $R^5$ being an alkyl group and with $R^{17}$ being a sterol side chain. Compounds with $R^{17}$ being the cholesterol side chain are still more preferred. Especially preferred as such an "dual antagonist" is 2-[(4aα-methyl-4a-homo-3α,5-methano-4-oxa-5α-cholest-8-en-3β-yl)oxy]ethanol.

A further object of the present invention are pharmaceutical compositions comprising one or more compounds of the general formula I as active substances. The compositions may further comprise pharmaceutically acceptable excipients well known in the art like carriers, diluents, absorption enhancers, preservatives, buffers, agents for adjusting the osmotic pressure, tablet disintegrating agents and other ingredients which are conventionally used in the art.

Examples of solid carriers are magnesium carbonate, magnesium stearate, dextrin, lactose, sugar, talc, gelatin, pectin, tragacanth, methylcellulose, sodium carboxymethyl cellulose, low melting waxes and cacao butter.

Liquid compositions include sterile solutions, suspensions and emulsions. Such liquid compositions may be suitable for injection or for use in connection with ex *vivo and in vitro* fertilization. The liquid compositions may contain other ingredients which are conventionally used in the art, some of which are mentioned in the list above. Further, a composition for transdermal administration of a compound of this invention may be provided in the form of a patch and a composition for nasal administraton may be provided in the form of a nasal spray in liquid or powder form.

The dose of a compound of the invention to be used will be determined by a physician and will depend among several factors on the particular compound employed, on the route of administration and on the purpose of the use. In general, the compositions of the invention are prepared by intimately bringing into association the active compound with the liquid or solid auxiliary ingredients and then, if necessary, shaping the product into the desired formulation.

Usually, not more than 1000 mg, preferably not more than 100 mg, and in some preferred instances not more than 10 mg of a compound of formula I is to be administered to mammals, e.g. to humans, per day.

The present invention also relates to the use of the compounds of the general formula II

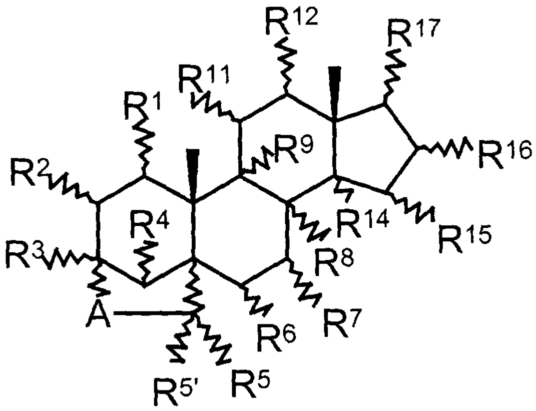

**II**

wherein

| | |
|---|---|
| $R^3$ | designates a hydrogen atom, a hydroxy group or a 2-hydroxyethoxy group, |
| A | designates an oxygen atom or a NH group, |
| $R^5$ | designates a hydrogen atom, a hydroxy group, an $C_1$-$C_8$ linear or branched alkyl or alkenyl group, a $C_6$-$C_{10}$ aryl group or together with $R^{5'}$ an oxo group, |
| $R^{5'}$ | designates a hydrogen atom or together with $R^{5'}$ an oxo group, and |
| $R^1$, $R^2$, $R^4$, $R^6$-$R^9$, $R^{11}$, $R^{12}$ and $R^{14}$-$R^{17}$ | designate any substituent, for the preparation of a meiosis-regulating medicament. |

Examples for $R^1$, $R^2$, $R^4$, $R^6$-$R^9$, $R^{11}$, $R^{12}$ and $R^{14}$-$R^{17}$ are: hydrogen, $C_1$-$C_8$ linear or branched alkyl or alkenyl, $C_6$-$C_{10}$ aryl, optionally substituted phenyl, cyano, $C_1$-$C_6$-hydroxyalkyl, hydroxy, halogen, $C_1$-$C_6$-perfluoralkyl, carbaldehyde, oxime derived from a carbaldehyde, carboxylic acid, ester with a $C_1$-$C_6$-alcohol, oxo, optionally substituted alkoxy, acyloxy, sulphonyloxyand phosphonyloxy. Further examples are described in the patent applications WO 96/00235, WO 96/27658, WO 97/00884 and WO 98/28323. These compounds have a meiosis-regulating effect. Activation of meiosis is determined as germinal vesicle breakdown as described in example 13. Inhibition of meiosis is tested as described in example 14. Compounds of the general formula II either activate meiosis by at least 40% or inhibit meiosis by at least 40%.

Preferred compounds of formula II are such wherein

$R^3$ designates a hydrogen atom, a hydroxy group or a 2-hydroxyethoxy group,
A designates an oxygen atom or a NH group,
$R^5$ designates a hydrogen atom, a hydroxy group, an $C_1$-$C_8$ linear or branched alkyl or alkenyl group, a $C_6$-$C_{10}$ aryl group or together with $R^{5'}$ an oxo group,
$R^{5'}$ designates a hydrogen atom or together with $R^5$ an oxo group,
$R^7$ designates a hydrogen atom or together with $R^8$ an additional bond,
R8 designates a hydrogen atom or together with R7 or together with R9 or together with $R^{14}$ an additional bond,
$R^9$ designates a hydrogen atom or together with $R^8$ an additional bond,
$R^{14}$ designates a hydrogen atom or together with $R^8$ or together with $R^{15}$ an additional bond,
$R^{17}$ a $C_1$-$C_{10}$ linear or branched optionally substituted alkyl or alkenyl group, an optionally substituted $C_8$-$C_{12}$ arylalkyl group, a $C_1$-$C_{10}$ linear or branched optionally substituted alkoxy group or an optionally substituted $C_6$-$C_{10}$ arylalkoxy group and
R1, R2, R4, R6, R11, R12, R15 and R16 designate a hydrogen atom. These preferred compounds are identical to the compounds of the general formula I.

Other preferred compounds of formula II are such wherein $R^3$ designates a hydrogen atom or a hydroxy group.

Other preferred compounds of formula II are such wherein $R^5$ and $R^{5'}$ designate together an oxo group.

Other preferred compounds of formula II are such wherein $R^{17}$ designate a $C_1$-$C_{10}$ linear or branched optionally substituted alkyl or alkenyl group or a $C_1$-$C_{10}$ linear or branched optionally substituted alkoxy group.

The compounds of the present invention influence the meiosis in oocytes as well as in male germ cells. The compounds of the general formula I and II are promising as new fertility-regulating agents without the usual side effects on the somatic cells which are known from the hitherto used hormonal contraceptives which are based on estrogens and/or gestagens.

In this connection it is important to notice, that the biosynthesis of progesterone in cultured human granulosa cells (somatic cells of the follicle) is not affected by the presence of a meiosis regulating substance whereas the estrogens and gestagens used in the hitherto used hormonal contraceptives do have an adverse effect on the biosynthesis of progesterone.

Contraception in females can be achieved by administration of a compound of the invention which inhibits the meiosis, so that no mature oocytes are produced. Similarly, contraception in males can be achieved by administration of a compound of the invention which inhibits the meiosis, so that no mature sperm cells are produced.

In a further aspect, the present invention relates to a method of regulating meiosis comprising administering to a subject in need of such a regulation an effective amount of one or more compounds of the general formula I or II.

The route of administration of compositions containing a compound of the invention may be any route which effectively transports the active compound to its site of action.

Thus, when the compounds of this invention are to be administered to a mammal, they are conveniently provided in the form of a pharmaceutical composition which comprises at least one compound of the invention in connection with a pharmaceutically acceptable carrier. For oral use, such compositions are preferably in the form of capsules or tablets.

When used as a contraceptive, the compounds of the invention will either have to be administered continuously or cyclically.

The compounds according to this invention can be synthesized analogously to the preparation of known compounds. Hence, synthesis of the compounds of this invention can follow the well established synthetic pathways described in the comprehensive sterol and steroid literature. The following books can be used as the key source in the synthesis: L.F. Fieser & M. Fieser: Steroids: Reinhold Publishing Corporation, NY 1959; Rood's Chemistry of Carbon Compounds (editor: S. Coffrey): Elsevier Publishing Company, 1971; and especially Dictionary of Steroids (editors: R. A. Hill; D.N. Kirk; H.L.J. Makin and G.M. Murphy): Chapman & Hall. The last one contains an extensive list of citations to the original papers covering the period up to 1990.

Particularly, the compounds of the present invention are synthesized according to the following general procedures:

The steroids that are used as starting materials are commercially available or can be synthesized according to known procedures:

- 5β-cyano-compounds: described in EP 0 957 108
- lichesterol: *J. Chem. Soc. Perkin Trans. I* (1981), 2125
- (20S)-20-hydroxymethyl-pregn-4-en-3-one: commercially available
- testosterone: commercially available

In the following only the syntheses in the 8-series or in the saturated series are described. The derivatives in the other double bond isomer series can be synthesized from the corresponding starting materials in an analogous way.

The lactones of formula 3 can be prepared by the following route. Starting from 5-cyano-5 -cholest-8-en-3 -ol 1 the cyano group is reduced to the corresponding aldehyde with diisobutylaluminum hydride. This aldehyde cyclyses immediately to the lactol of formula 2. Oxidation with chromiumtrioxide-pyridine-complex gives the desired lactones of formula 3.

Scheme 1:

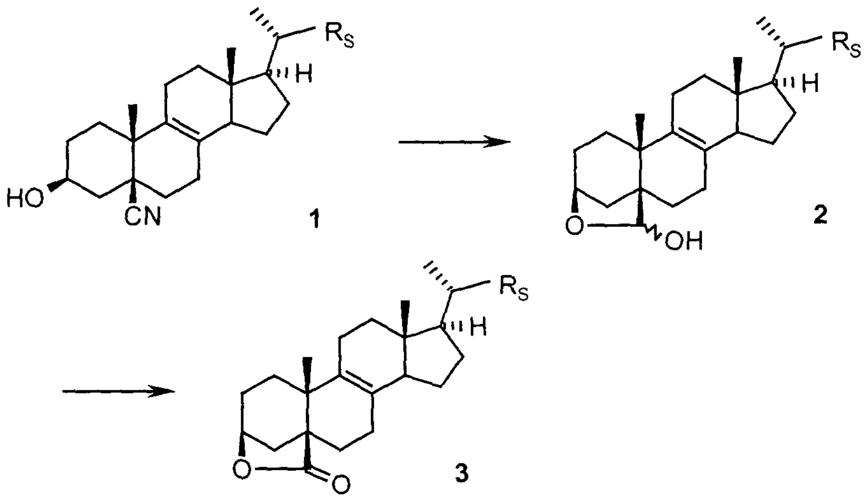

1

2

3

The reactions can be carried out in the presence of different steroidal side chains ($R_S$) like cholesterol, ergosterol, sitosterol or stigmasterol side chain.

The hydroxylactams of formula **5** are easily synthesized from cyanoketone **4** by a basic hydrolysis analogously to a literature procedure.

scheme 2:

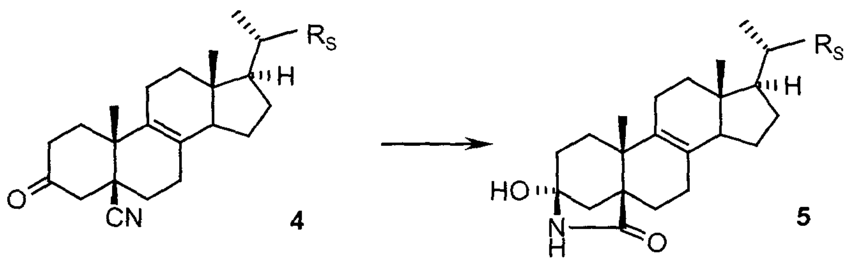

4

5

Hemiketals of formula **9** can be prepared by the following route. Starting from cyanoketone **4** the carbonyl group is protected as a cyclic ketal. The reduction of the cyano group to aldehyde **7** can be accomplished with diisobutylalumnum hydride.

This aldehyde can be reacted with different Grignard reagents to give secondary alcohols of formula **8**. These can be transformed to the hemiketals of formula **9** by the action of the acidic ion exchanger amberlyste 15.

Scheme 3:

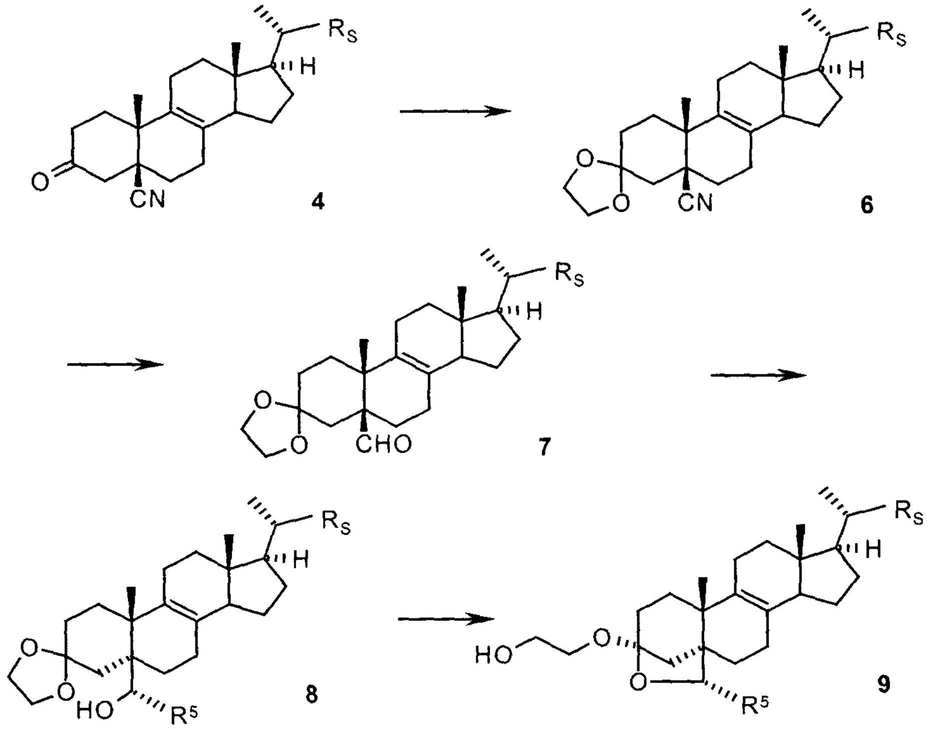

4

6

7

8

9

Side chain derivatives in the lactone series can be obtained on the following route. The hydroxy group in enone **10** is protected with triisoproplylsilyl chloride. The cyanogroup in position 5 can be introduced as has been described before. Cyanoketone **12** can be reduced with L-selectride to give selectively the axial alcohol **13**. This can be transformed to the lactol **14** as described in scheme 1. Further steps are deprotection and tosylation in the side chain to give tosylate **16**, which can be coupled with different Grignard reagents under copper catalysis to give lactons of formula **17**.

Scheme 4:

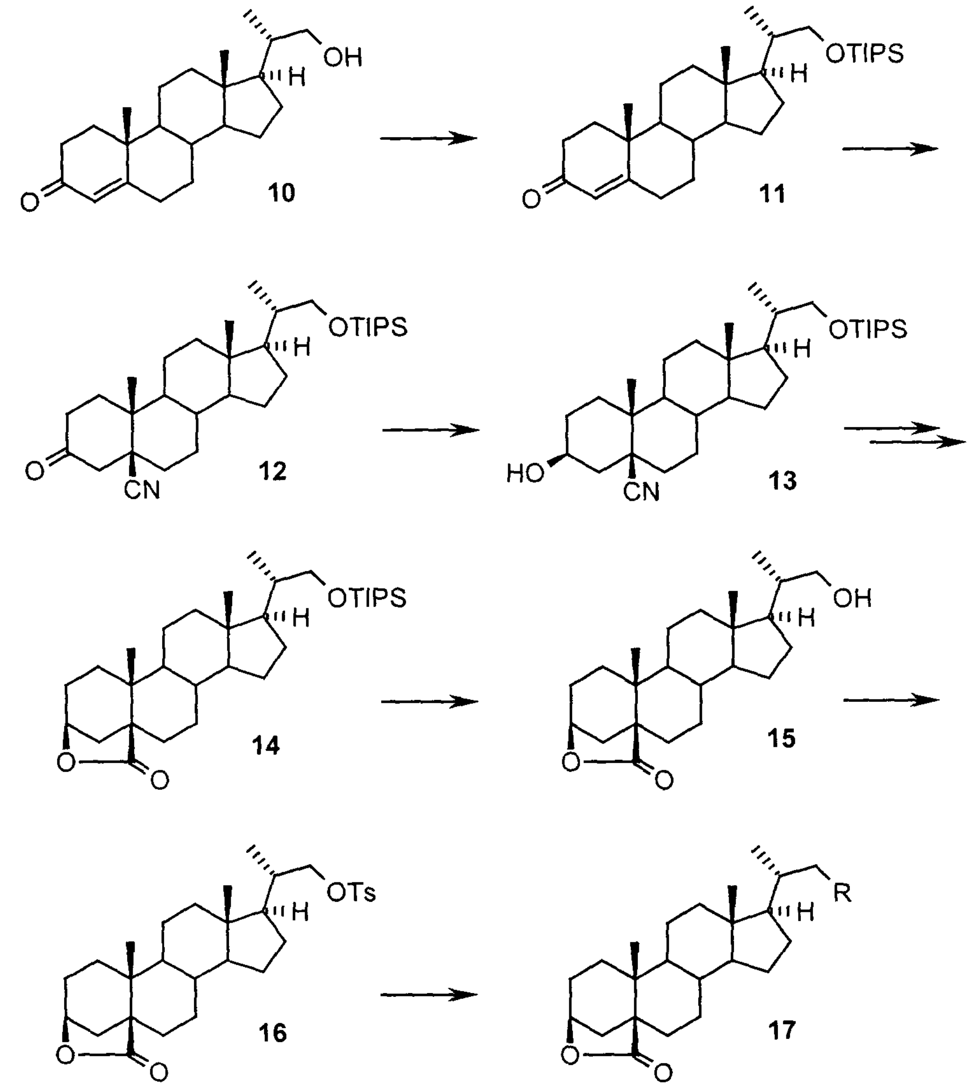

10 11 12 13 14 15 16 17

Side chain derivatives in the hemiketal series can be obtained in a similar manner. Starting from ergosterol derivatve **6** (Rs = ergosterol side chain) the double bond in the side chain can be cleaved by ozonolysis. Reductive work up gives alcohol **18.** The primary alcohol can be tosylated and coupled with different Grignard reagents to give side chain derivatives of formula **20.** These can be further transformed to the desired hemiketals **23** as described above for the normal sterol side chains.

Scheme 5:

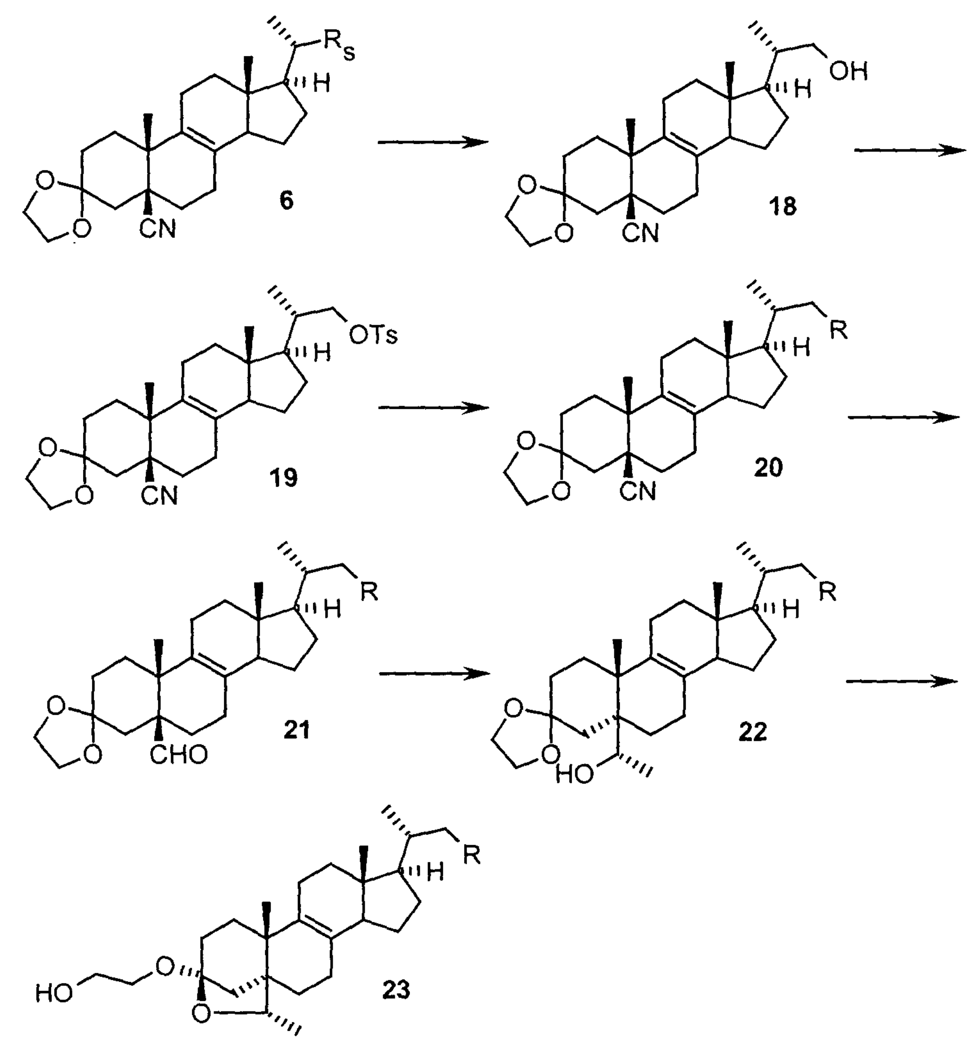

6 18 19 20 21 22 23

The 17-alkoxy derivatives in the lactone series can be obtained by the following sequence. Starting from testosterone **24** the hydroxyl group is protected with triisopropylsilylchloride. Then the lactone can be constructed as described above.
After deprotection of the side chain, alcohol **30** can be alkylated with different alkylhalides to give ethers of formula **31.**

Scheme 6:

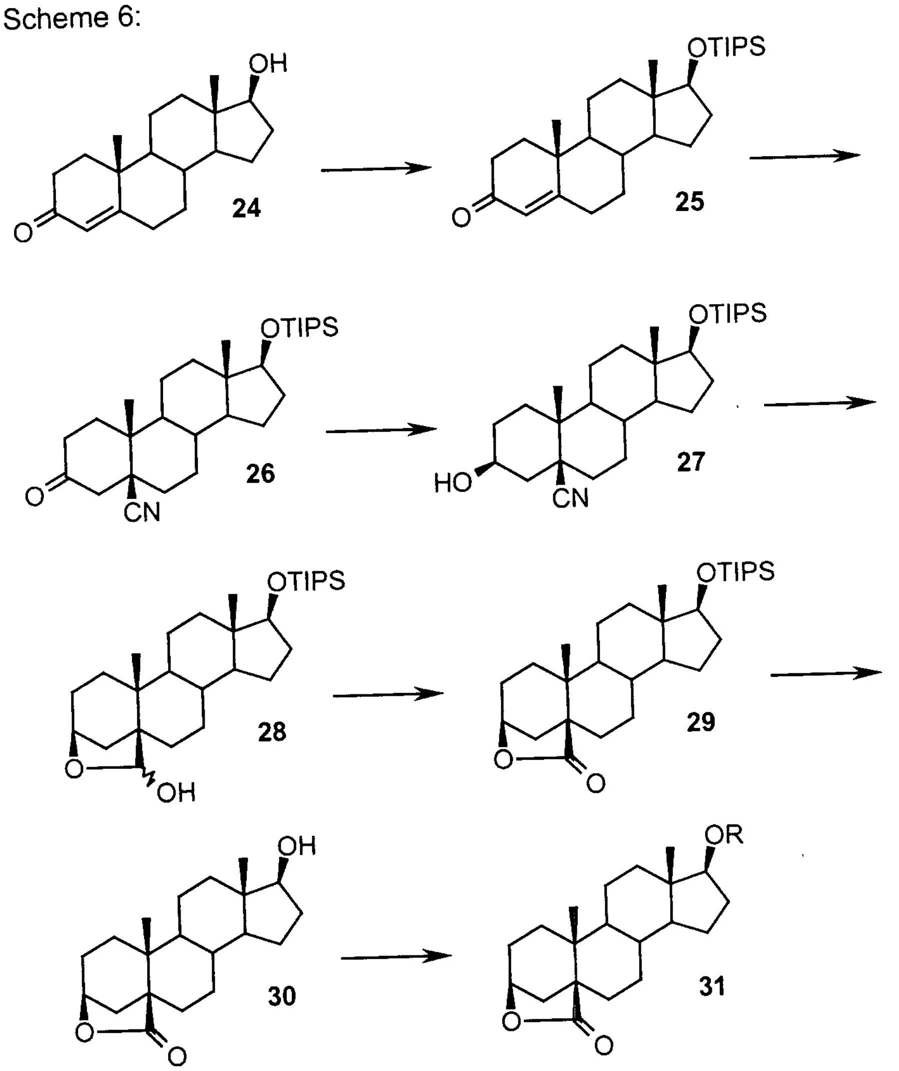

24 25

26 27

28 29

30 31

The present invention is further illustrated by the following examples:

**Example 1: 5β-cholest-8-ene-5,3β-carbolactone**

a) 5β-cholest-8-ene-5,3β-carbolactol

To a solution of 90 mg 5-cyano-5β-cholest-8-en-3β-ol in 3 ml toluene were added 1.83 ml of a diisobutylaluminum hydride solution [1.2 M in toluene] at -10°C. The solution was stirred for 45 minutes and 1.4 ml of sulfuric acid [2 N] were added. The resulting mixture was refluxed for one hour. After cooling to room temperature the residue was diluted with dichloromethane, washed with water and brine and dried over anhydrous sodium sulfate. After filtration and evaporation of the solvents the product was purified by column chromatography to give 77 mg 5β-cholest-8-ene-5,3β-carbolactol.
$^1$H-NMR ($CDCl_3$): δ = 0.64 (s, 3H, H-18); 0.87 (2xd, J=7 Hz, 6H, H-26/27); 0.94 (d, J = 7 Hz, 3H, H-21); 4.43 (t, J=5 Hz, 1H, H-3); 5.20 (d, J=5 Hz, 1H, H-5')

b) 5β-cholest-8-ene-5,3β-carbolactone

To a solution of 77 mg 5β-cholest-8-ene-5,3β-carbolactol in 9 ml dichloromethane were added 359 mg chromiumtrioxide pyridine complex at room temperature. The mixture was stirred for two hours. The residue was diluted with dichloromethane, washed with water and brine and dried over anhydrous sodium sulfate. After evaporation of the solvents the product was purified by column chromatography to give 11 mg 5β-cholest-8-ene-5,3β-carbolactone as a

white solid.
$^1$H-NMR ($CDCl_3$): δ = 0.63 (s, 3H, H-18); 0.87 (2xd, J=7 Hz, 6H, H-26/27); 0.94 (d, J=7 Hz, 3H, H-21); 4.72 (t, J=5 Hz, 1H, H-3)

**Example 2: 5β-cholest-8(14)-ene-5,3β-carbolactone**

a) β-cholest-8(14)-ene-5,3β-carbolactol

90 mg 5-cyano-5β-cholest-8(14)-en-3β-ol were treated with diisobutylaluminum hydride as described in example 1a. After work up and column chromatography 70 mg 5β-cholest-8(14)-ene-5,3β-carbolactol were obtained.
$^1$H-NMR ($CDCl_3$): δ = 0.79 (s, 3H); 0.85 (s, 3H); 0.86 (2xd, J=7 Hz, 6H, H-26/27); 0.92 (d, J=7 Hz, 3H, H-21); 4.44 (t, J=5 Hz, 1H, H-3); 5.20 (s, 1H, H-5')

b) 5β-cholest-8(14)-ene-5,3β-carbolactone

60 mg 5β-cholest-8(14)-ene-5,3β-carbolactol were treated with chromiumtrioxide pyridine complex as described in example 1b. After work up and column chromatography 15 mg 5β-cholest-8(14)-ene-5,3β-carbolactone were obtained as a white solid.
$^1$H-NMR ($CDCl_3$): δ = 0.89 (2xs, 2xd, J=7 Hz, 12H, H-18/19/26/27); 0.95 (d, J=7 Hz, 3H, H-21); 2.55 (m, 2H, H-4); 4.72 (t, J=5 Hz, 1H, H-3)

**Example 3: 3α-hydroxy-5β-cholest-8-ene-5,3β-carbolactame**

200 mg 5-cyano-5β-cholest-8-en-3-one were added to a solution of 1.27 g potassium hydroxide in 32 ml ethanol. After stirring for 24 hours at room temperature the solution was poured into hydrochloric acid [1 M] and the aqueous layer was extracted with dichloromethane. The organic layer was washed with brine, dried over sodium sulfate, filtered and concentrated under reduced pressure. The residue was purified by column chromatography to give 83 mg 3α-hydroxy-5β-cholest-8-ene-5,3β-carbolactame as a white solid.
$^1$H-NMR ($CDCl_3$): δ = 0,63 (s, 3H, H-18); 0,87 (2x d,J=7 Hz, 6H, H-26/27); 0,94 (d, J= 7 Hz, 3H, H-21); 1.07 (s, 3H, H-19); 2.88 (s, 1H, OH); 5.72 (s, 1H, NH)

**Example 4: 3α-hydroxy-5β-cholest-8(14)-ene-5,3β-carbolactame**

50 mg 5-cyano-5β-cholest-8(14)-en-3β-one were treated with potassium hydroxide in ethanol as described in example 3. After work up and column chromatography 34 mg 3α-hydroxy-5β-cholest-8(14)-ene-5,3β-carbolactame were obtained as a white solid.
$^1$H-NMR ($CDCl_3$): δ = 0,85 (2xs, 6H, H-18/19); 0,86 (2x d,J=7 Hz, 6H, H-26/27); 0,94 (d, J= 7 Hz, 3H, H-21); 3.38 (bs, 1H, OH); 6.10 (s, 1H, NH)

**Example 5: 2-[(4aα-methyl-4a-homo-3α,5-methano-4-oxa-5α-cholest-8-en-3β-yl)oxy]ethanol**

a) 5-cyano-3,3-(ethylenedioxy)-5β-cholest-8-ene

A mixture of 2.98 g 5-cyano-5β-cholest-8-en-3β-one, 3.34 ml ethylene glycol and 83 mg p-toluenesulfonic acid in 69 ml toluene was refluxed for one hour. The mixture was diluted with ethyl acetate, washed with water, saturated sodium bicarbonate solution and brine. The organic layer was dried over sodium sulfate and filtered. Removal of the solvents gave 3.41 g 5-cyano-3,3-(ethylenedioxy)-5β-cholest-8-ene, which was used without further purification.
$^1$H-NMR ($CDCl_3$): δ = 0,63 (s, 3H, H-18); 0,87 (2x d,J=7 Hz, 6H, H-26/27); 0,94 (d, J= 7 Hz, 3H, H-21); 1.29 (s, 3H, H-19); 3.90 - 4.08 (m, 4H, ketal)

b) 3,3-(ethylenedioxy)-5-formyl-5β-cholest-8-ene

A solution of 3.88 g g 5-cyano-3,3-(ethylenedioxy)-5β-cholest-8-ene in 236 ml toluene was treated with 62 ml diisobutylaluminumhydride solution [1.2 M, toluene] as described in example 1a. After hydrolysis with 48 ml sulfuric acid, work up and purification as described above 1.35 g 3,3-(ethylenedioxy)-5-formyl-5β-cholest-8-ene were isolated as a white solid.
$^1$H-NMR ($CDCl_3$): δ = 0,63 (s, 3H, H-18); 0,87 (2x d, J=7 Hz, 6H, H-26/27); 0,94 (d, J= 7 Hz, 3H, H-21); 1.09 (s, 3H, H-19); 3.85 - 3.97 (m, 4H, ketal)

c) (5'S)-3,3-(ethylenedioxy)-5-(1-hydroxyethyl)-5β-cholest-8-ene

0.27 ml of a methylmagnesium bromide solution [3 M, diethylether] were added to a solution of 200 mg 3,3-(ethylenedioxy)-5-formyl-5β-cholest-8-ene in 10 ml diethylether at -10 °C. The mixture was stirred for two hours at 0 °C and then poured into saturated ammonium chloride solution. The solution was extracted with ethyl acetate and the combined extracts were further washed with water and brine and then dried over sodium sulfate. The solution was filtered and evaporated in vacuo. The crude product was purified by chromatography to yield 142 mg (5'S)-3,3-(ethylenedioxy)-5-(1-hydroxyethyl)-5β-cholest-8-ene as a white solid.
$^1$H-NMR ($CDCl_3$): δ = 0,61 (s, 3H, H-18); 0,87 (2x d, J=7 Hz, 6H, H-26/27); 0,91 (d, J= 7 Hz, 3H, H-21); 1.08 (s, 3H, H-19); 1.20 (d, J=7Hz, 3H); 3.77 (m, 1H, H-5'); 3.85 - 3.97 (m, 4H, ketal)

d) 2-[(4aα-methyl-4a-homo-3α,5-methano-4-oxa-5α-cholest-8-en-3β-yl)oxy] ethanol

A mixture of 63 mg (5'S)-3,3-(ethylenedioxy)-5-(1-hydroxyethyl)-5β-cholest-8-ene, 3 ml acetone and 15 mg amberlyste 15 was stirred for two hours at room temperature. After filtration the solvent was removed in vacuo and the residue purified by chromatography to yield 39 mg 2-[(4aα-methyl-4a-homo-3α,5-methano-4-oxa-5α-cholest-8-en-3β-yl)oxy]ethanol as a white solid.
$^1$H-NMR ($CDCl_3$): δ = 0,61 (s, 3H, H-18); 0,87 (2x d, J=7 Hz, 6H, H-26/27); 0,93 (d, J= 7 Hz, 3H, H-21); 0.97 (s, 3H, H-19); 1.12 (d, J=7Hz, 3H); 3.65 - 4.12 (m, 5H, ketal, H-5')

**Example 6: 2-[(4aα-ethyl-4a-homo-3α,5-methano-4-oxa-5α-cholest-8-en-3β-yl)oxy]ethanol**

a) 3,3-(ethylenedioxy)-(5'S)-5-(1-hydroxypropyl)-5β-cholest-8-ene

100 mg 3,3-(ethylenedioxy)-5-formyl-5β-cholest-8-ene were treated with ethylmagnesium bromide analogously to example 5c. After work up and chromatography 59 mg 3,3-(ethylenedioxy)-(5'S)-5-(1-hydroxypropyl)-5β-cholest-8-ene were isolated as an oil.
$^1$H-NMR ($CDCl_3$): δ= 0,59 (s, 3H, H-18); 0,85 (2x d, J=7 Hz, 6H, H-26/27); 0,91 (d, J= 7 Hz, 3H, H-21); 1.03 It, J= 7Hz, 3H); 1.06 (s, 3H, H-19); 3.80 (t, J= 8 Hz, 1H, H-5'); 3.92 - 4.02 (m, 4H, ketal)

b) 2-[(4aα-ethyl-4a-homo-3α,5-methano-4-oxa-5α-cholest-8-en-3β-yl)oxy]ethanol

32 mg 3,3-(ethylenedioxy)-(5'S)-5-(1-hydroxypropyl)-5β-cholest-8-ene were treated with amberlyste 15 as described in example 5d. After filtration the solvent was removed in vacuo and the residue purified by chromatography to yield 25 mg 2-[(4aα-ethyl-4a-homo-3α,5-methano-4-oxa-5α-cholest-8-en-3β-yl)oxy]ethanol.
$^1$H-NMR ($CDCl_3$): δ= 0,62 (s, 3H, H-18); 0,86 (2x d, J=7 Hz, 6H, H-26/27); 0,93 (d, J= 7 Hz, 3H, H-21); 0.99 (s, 3H, H-19); 1.03 (t, J= 7Hz, 3H); 3.80 (t, J= 8 Hz, 1H, H-5'); 3.65 - 3.70 (m, 5H, ketal, H-5')

**Example 7: 2-[(4aα-methyl-4a-homo-3α,5-methano-4-oxa-24-nor-5α-cholest-8-en-3β-yl)oxy]ethanol**

a) 5-cyano-5β-ergosta-8,22-dien-3-one

A solution of 1.90 g lichesterol in 19 ml toluene and 3.8 ml cyclohexanone was refluxed for 10 minutes at a dean-stark-trap before a solution of 0.5 g of aluminum tris-isopropylate in 3.8 ml toluene was added. The reaction mixture was refluxed for further 30 minutes, cooled to room temperature and then poured into sulfuric acid [2N]. The solution was extracted with ethyl acetate and the combined extracts were further washed with saturated sodium bicarbonate solution and brine and then dried over anhydrous sodium sulfate. The solution was filtered and evaporated in vacuo. The crude product was purified by chromatography to yield 1.54 g ergosta-4,8,22-trien-3-one as a white solid.
11.73 ml diethylaluminum cyanide solution [1N, toluene] were added to a solution of 1.54 g ergosta-4,8,22-trien-3-one in 47 ml tetrahydofuran at 0°C. The reaction mixture was stirred for 3.5 hours at room temperature. 20 ml of aqueous sodium hydroxide solution were aded dropwise under ice-cooling. The mixture was extracted with ethyl acetate and the combined extracts washed brine and then dried over sodium sulfate. The solution was filtered and evaporated in vacuo. The crude product was purified by chromatography to yield 0.53 g 5-cyano-5β-ergosta-8,22-dien-3-one as pale yellow solid (besides 0.90 g 5-cyano-5α-ergosta-8,22-dien-3-one).
$^1$H-NMR ($CDCl_3$): δ = 0.70 (s, 3H, H-18); 0.84 (2x d, J=7 Hz, 6H, H-26/27); 0.92 (d, J= 7 Hz, 3H, H-21); 1.04 (d, J=7 Hz,3H, H28); 1.43 (s, 3H, H-19); 2.64 (s, 2H, H-4); 5.21 (m, 2H, H-22/23)

b) 5-cyano-3,3-(ethylenedioxy)-5β-ergosta-8,22-diene

A mixture of 0.53 g 5-cyano-5β-ergosta-8,22-dien-3-one, 0.57 ml ethylene glycol and 15 mg p-toluenesulfonic acid in 12 ml toluene was refluxed for two hours. The mixture was diluted with ethyl acetate, washed with water, saturated sodium bicarbonate solution and brine. The organic layer was dried over sodium sulfate and filtered. Removal of the solvents gave 0.52 g 5-cyano-3,3-(ethylenedioxy)-5β-ergosta-8,22-diene, which was used without further purification.

$^1$H-NMR ($CDCl_3$): 6= 0.65 (s, 3H, H-18); 0.84 (2x d, J=7 Hz, 6H, H-26/27); 0.92 (d, J= 7 Hz, 3H, H-21); 1.04 (d, J=7 Hz,3H, H28); 1.30 (s, 3H, H-19); 3.90-4.08 (m, 4H, ketal); 5.20 (m, 2H, H-22/23)

c) (20R)-5-cyano-3,3-(ethylenedioxy)-20-(hydroxymethyl)-5β-pregn-8-ene

A solution of 0.52 g 5-cyano-3,3-(ethylenedioxy)-5β-ergosta-8,22-diene in 24 ml dichloromethane, 8.4 ml methanol and 0.3 ml pyridine was cooled to -70 °C. A ozone/oxygene-mixture (1:4) was passed through the solution for 24 minutes. Then 170 mg sodium borohydride were added and the stirred reaction mixture was allowed to warm to 0°C and poured into saturated aqueous ammonium chloride solution. The solution was extracted with ethyl acetate and the combined extracts were further washed with water and brine and then dried over sodium sulfate. The solution was filtered and evaporated in vacuo. The crude product was purified by chromatography to yield 182 mg (20R)-5-cyano-3,3-(ethylenedioxy)-20-(hydroxymethyl)-5β-pregn-8-ene as a white foam.

$^1$H-NMR ($CDCl_3$): δ = 0.67 (s, 3H, H-18); 1.06 (d, J=7 Hz, 3H, H-21); 1.30 (s, 3H, H-19); 3.38 (dd, J = 11Hz, 7Hz, 1H, H-22) 3.65 (dd, J = 11Hz, 3Hz, 1H, H-22) 3.90-4.08 (m, 4H, ketal)

d) (20R)-5-cyano-3,3-(ethylenedioxy)-20-(p-toluenesulfonyloxymethyl)-5β-pregn-8-ene

197 mg p-toluenesulfonic chloride were added to a solution of 165 mg (20R)-5-cyano-3,3-(ethylenedioxy)-20-(hydroxymethyl)-5β-pregn-8-ene in 3.1 ml pyridine at room temperature. The mixture is stirred for 72 hours and then poured into hydrochloric acid [0.5 N]. The solution was extracted with ethyl acetate and the combined extracts were washed with water and brine, dried over sodium sulfate, filtered and evaporated in vacuo to yield 199 mg of crude (20R)-5-cyano-3,3-(ethylenedioxy)-20-(p-toluenesulfonyloxymethyl)-5β-pregn-8-ene, which was used without further purification.

$^1$H-NMR ($CDCl_3$): δ = 0.62 (s, 3H, H-18); 1.01 (d, J=7 Hz, 3H, H-21); 1.30 (s, 3H, H-19); 2.46 (s, 3H); 3.75-4.08 (m, 6H, ketal, H-22); 7.37 (m, 2H, arom.); 7.80 (m, 2H, arom.)

e) 5-cyano-3,3-(ethylenedioxy)-24-nor-5β-cholest-8-ene

A isobutylgrignard reagent was prepared from 3.64 ml isobutylbromide and 2.17 g magnesium in 30 ml tetrahydrofuran. 4.46 ml of a $Li_2CuCl_3$-solution [0.1 M in THF] was added to the grignard solution at -30°C and the resulting mixture was stirred for 30 minutes. A solution of 600 mg of (20R)-5-cyano-3,3-(ethylenedioxy)-20-(p-toluenesulfonyloxymethyl)-5β-pregn-8-ene in 15 ml tetrahydrofuran was added at -30°C and the resulting mixture was allowed to warm to room temperature overnight. It was poured into saturated ammonium chloride solution, extracted with ethyl acetate and the combined extracts were washed with water and brine, dried over sodium sulfate and filtered. After evaporation of the solvents the residue was purified by column chromatography to give 298 mg 5-cyano-3,3-(ethylenedioxy)-24-nor-5β-cholest-8-ene.

$^1$H-NMR ($CDCl_3$): δ = 0.63 (s, 3H, H-18); 0.85 (2d, 6H, H-25/26); 0.92 (d, J = 7 Hz, 3H, H-21); 1.28 (s, 3H, H-19); 3.90-4.08 (m, 4H, ketal)

f) 3,3-(ethylenedioxy)-5-formyl-24-nor-5β-cholest-8-ene

274 mg mg 5-cyano-3,3-(ethylenedioxy)-24-nor-5β-cholest-8-ene were reduced with 5.18 ml disobutylaluminum hydride solution as described in example 1a to give 115 mg 3,3-(ethylenedioxy)-5-formyl-24-nor-5β-cholest-8-ene after column chromatography.

$^1$H-NMR ($CDCl_3$): δ = 0.63 (s, 3H, H-18); 0.85 (2d, 6H, H-25/26); 0.92 (d, J=7 Hz, 3H, H-21); 1.10 (s, 3H, H-19); 3.85-3.98 (m, 4H, ketal); 9.99 (s, 1H, aldehyde)

g) (5'S)-(3,3-ethylenedioxy)-5-(1-hydroxyethyl)-24-nor-5β-cholest-8-ene

110 mg 3,3-(ethylenedioxy)-5-formyl-24-nor-5β-cholest-8-ene were treated with methylmagnesium bromide as described in example 5c. After work up and chromatography 72 mg (5'S)-(3,3-ethylenedioxy)-5-(1-hydroxyethyl)-24-nor-5β-cholest-8-ene were obtained as an oil.

$^1$H-NMR ($CDCl_3$): δ = 0.61 (s, 3H, H-18); 0.85 (2d, 6H, H-25/26); 0.89 (d, J=7 Hz, 3H, H-21); 1.07 (s, 3H, H-19); 1.19 (d, J=7 Hz, 3H); 3.75 (m, 1H, H-5'); 3.85-4.05 (m, 4H, ketal)

h) 2-[(4aα-methyl-4a-homo-3α,5-methano-4-oxa-24-nor-5α-cholest-8-en-3β-yl)oxy]ethanol

70 mg mg (5'S)-(3,3-ethylenedioxy)-5-(1-hydroxyethyl)-24-nor-5β-cholest-8-ene were treated with amberlyste 15 as described in example 5d. After chromatography 57 mg 2-[(4aα-methyl-4a-homo-3α,5-methano-4-oxa-24-nor-5α-cholest-8-en-3β-yl)oxy]ethanol were obtained as a white foam.

$^1$H-NMR ($CDCl_3$): δ = 0.63 (s, 3H, H-18); 0.85 (2d, 6H, H-25/26); 0.92 (d, J=7 Hz, 3H, H-21); 0.97 (s, 3H, H-19); 1.14 (d, J=7 Hz, 3H); 3.85-4.15 (m, 5H, ketal, H-5')

**Example 8: 2-[(20R)-(21-cyclohexyl-4aα,20-dimethyl-4a-homo-3α,5-methano-4-oxa-5α-pregn-8-en-3β-yl)oxy] ethanol**

a) (20R)-21-cyclohexyl-5-cyano-3,3-(ethylenedioxy)-20-methyl-5β-pregn-8-ene

190 mg (20S)-5-cyano-3,3-(ethylenedioxy)-20-(p-toluenesulfonyloxymethyl)-5β-pregn-8-ene were treated with cyclohexylmagnesium bromide analogously to example 6e. After work up and chromatography 68 mg (20R)-21-cyclohexyl-5-cyano-3,3-(ethylenedioxy)-20-methyl-5β-pregn-8-ene were obtained as an oil.

$^1$H-NMR ($CDCl_3$): δ = 0.64 (s, 3H, H-18); 0.90 (d, J=7 Hz, 3H, H-21); 1.28 (s, 3H, H-19); 3.90-4.08 (m, 4H, ketal)

b) (20R)-21-cyclohexyl-3,3-(ethylenedioxy)-5-formyl-20-methyl-5β-pregn-8-ene

215 mg (20R)-21-cyclohexyl-5-cyano-3,3-(ethylenedioxy)-20-methyl-5β-pregn-8-ene were reduced with 3.84 ml disobutylaluminum hydride solution as described in example 1a to give 88 mg (20R)-21-cyclohexyl-3,3-(ethylenedioxy)-5-formyl-20-methyl-5β-pregn-8-ene after column chromatography.

$^1$H-NMR ($CDCl_3$): δ = 0.63 (s, 3H, H-18); 0.91 (d, J=7 Hz, 3H, H-21); 1.10 (s, 3H, H-19); 3.85-3.98 (m, 4H, ketal); 9.98 (s, 1H, aldehyde)

c) (5'S, 20R)-21-cyclohexyl-3,3-(etylenedioxy)-5-(1-hydroxyethyl)-20-methyl-5β-pregn-8-ene

78 mg (20R)-21-cyclohexyl-3,3-(ethylenedioxy)-5-formyl-20-methyl-5β-pregn-8-ene were treated with methylmagnesium bromide as described in example 5c. After work up and chromatography 57 mg (5'S, 20R)-21-cyclohexyl-3,3-(ethylenedioxy)-5-(1-hydroxyethyl)-20-methyl-5β-pregn-8-ene were obtained as an oil.

$^1$H-NMR ($CDCl_3$): δ = 0.61 (s, 3H, H-18); 0.89 (d, J=7 Hz, 3H, H-21); 1.07 (s, 3H, H-19); 1.19 (d, J=7 Hz, 3H); 3.76 (m, 1H, H-5'); 3.85-4.05 (m, 4H, ketal)

d) 2-[(20R)-(21-cyclohexyl-4aα,20-dimethyl-4a-homo-3α,5-methano-4-oxa-5α-pregn-8-en-3β-yl)oxy]ethanol

50 mg (5'S, 20R)-21-cyclohexyl-3,3-(etylenedioxy)-5-(1-hydroxyethyl)-20-methyl-5β-pregn-8-ene were treated with amberlyste 15 as described in example 5d. After chromatography 43 mg 2-[(20R)-(21-cyclohexyl-4aα,20-dimethyl-4a-homo-3α,5-methano-4-oxa-5α-pregn-8-en-3β-yl)oxy]ethanol were obtained as a white foam.

$^1$H-NMR ($CDCl_3$): δ = 0.63 (s, 3H, H-18); 0.92 (d, J=7 Hz, 3H, H-21); 0.96 (s, 3H, H-19); 1.14 (d, J=7 Hz, 3H); 3.65-4.15 (m, 5H, ketal, H-5')

**Example 9: 2-[(20R)-(4aα,20-dimethyl-4a-homo-3α,5-methano-4-oxa-21-phenyl-5α-pregn-8-en-3β-yl)oxy] ethanol**

a) (20R)-5-cyano-3,3-(ethylenedioxy)-20-methyl-21-phenyl-5β-pregn-8-ene

600 mg (20S)-5-cyano-3,3-(ethylenedioxy)-20-(p-toluenesulfonyloxymethyl)-5β-pregn-8-ene were treated with phenylmagnesium bromide analogously to example 6e. After work up and chromatography 169 mg (20R)-5-cyano-3,3-(ethylenedioxy)-20-methyl-21-phenyl-5β-pregn-8-ene were obtained as an oil.

$^1$H-NMR ($CDCl_3$): δ = 0.67 (s, 3H, H-18); 0.83 (d, J=7 Hz, 3H, H-21); 1.28 (s, 3H, H-19); 3.90-4.08 (m, 4H, ketal); 7.12-7.31 (m, 5H, arom.)

b) (20R)-3,3-(ethylenedioxy)-5-formyl-20-methyl-21-phenyl-5β-pregn-8-ene

165 mg (20R)-5-cyano-3,3-(ethylenedioxy)-20-methyl-21-phenyl-5β-pregn-8-ene were reduced with 2.99 ml disobutylaluminum hydride solution as described in example 1a to give 41 mg (20R)-3,3-(ethylenedioxy)-5-formyl-20-methyl-21-phenyl-5β-pregn-8-ene after column chromatography.

$^1$H-NMR ($CDCl_3$): δ = 0.67 (s, 3H, H-18); 0.85 (d, J=7 Hz, 3H, H-21); 1.10 (s, 3H, H-19); 3.85-3.98 (m, 4H, ketal); 7.12-7.31 (m, 5H, arom.); 10.00 (s, 1H, aldehyde)

c) (5'S, 20R)-3,3-(ethylenedioxy)-5-(1-hydroxyethyl)-20-methyl-21-phenyl-5β-pregn-8-ene

40 mg (20R)-3,3-(ethylenedioxy)-5-formyl-20-methyl-21-phenyl-5β-pregn-8-ene were treated with methylmagnesium bromide as described in example 5c. After work up and chromatography 19 mg (5'S, 20R)-3,3-(ethylenedioxy)-5-(1-hydroxyethyl)-20-methyl-21-phenyl-5β-pregn-8-ene were obtained as an oil.

$^1$H-NMR ($CDCl_3$): δ = 0.64 (s, 3H, H-18); 0.84 (d, J=7 Hz, 3H, H-21); 1.07 (s, 3H, H-19); 1.21 (d, J=7 Hz, 3H); 3.76 (m, 1H, H-5'); 3.85-4.05 (m, 4H, ketal); 7.12-7.30 (m, 5H, arom.)

d) 2-[(20R)-(4aα,20-dimethyl-4a-homo-3α,5-methano-4-oxa-21-phenyl-5α-pregn-8-en-3β-yl)oxy]ethanol

18 mg (5'S, 20R)-3,3-(ethylenedioxy)-5-(1-hydroxyethyl)-20-methyl-21-phenyl-5β-pregn-8-ene were treated with amberlyste 15 as described in example 5d. After chromatography 14 mg 2-[(20R)-(4aα,20-dimethyl-4a-homo-3α, 5-methano-4-oxa-21-phenyl-5α-pregn-8-en-3β-yl)oxy]ethanol were obtained as a white foam.

$^1$H-NMR ($CDCl_3$): δ = 0.66 (s, 3H, H-18); 0.85 (d, J=7 Hz, 3H, H-21); 0.98 (s, 3H, H-19); 1.14 (d, J=7 Hz, 3H); 3.65-4.15 (m, 5H, ketal, H-5'); 7.12-7.30 (m, 5H, arom.)

**Example 10: 24-nor-5β-cholestane-5,3β-carbolactone**

a) (20S)-20-(triisopropylsilyloxymethyl)-pregn-4-en-3-one

17.5 ml triisopylsilylchlorid were added to a solution of 20 g (20S)-20-(hydroxymethyl)-pregn-4-en-3-one, 9 g imidazole and 10 mg 4-DMAP in 200 ml dchloromethane at room temperature. The mixture was stirred for 20 hours and then poured into water. The product is extracted with diethylether, the combined extracts were washed with water and brine, dried over sodium sulfate and filtered to give 34.6 g of crude (20S)-20-(triisopropylsilyloxymethyl)-pregn-4-en-3-one which was used without further purification.

$^1$H-NMR ($CDCl_3$): δ = 0.75 (s, 3H, H-18); 1.02 (d, J=7 Hz, 3H, H-21); 1.07 (b, 18H, TIPS); 1.19 (s, 3H, H-19); 3.37 (dd, J=10 Hz, 7 Hz, 1H, H-22); 3.68 (dd, J=10 Hz, 3 Hz, 1H, H-22); 5.72 (s, 1H, H-4)

b) (20S)-5-cyano-20-(triisopropylsilyloxymethyl)-5β-pregnan-3-one

14 g (20S)-20-(triisopropylsilyloxymethyl)-pregn-4-en-3-one were treated with diethylaluminumcyanide solution analogously to example 7c. After work up and chromatography 7.6 g (20S)-5-cyano-20-(triisopropylsilyloxymethyl)-5β-pregnan-3-one were isolated besides 6.9 g of the corresponding 5α-product.

$^1$H-NMR ($CDCl_3$): δ = 0.71 (s, 3H, H-18); 1.02 (d, J=7 Hz, 3H, H-21); 1.07 (b, 18H, TIPS); 1.27 (s, 3H, H-19); 3.02 (d, J=16 Hz, 1H, H-4); 3.37 (dd, J = 10 Hz, 7 Hz, 1H, H-22); 3.65 (dd, J=10 Hz, 3 Hz, 1H, H-22

c) (20S)-5-cyano-20-(triisopropylsilyloxymethyl)-5β-pregnan-3β-ol

29.8 ml K-selectride solution [1 M, tetrahydrofuran] were added to a solution of 7.6 g (20S)-5-cyano-20-(triisopropylsilyloxymethyl)-5β-pregnan-3-one in 412 ml tetrahydrofuran at -72°C. The mixture was stirred for one hour at that temperature and then quenched with saturated ammonium chloride solution. After warming to room temperature the product is extracted with ethyl acetate, the combined extracts were washed with water and brine, dried over sodium sulfate, filtered and evaporated in vacuo. After column chromatography 7.12 g (20S)-5-cyano-20-(triisopropylsilyloxymethyl)-5β-pregnan-3β-ol were isolated.

$^1$H-NMR ($CDCl_3$): δ = 0.67 (s, 3H, H-18); 1.02 (d, J=7 Hz, 3H, H-21); 1.06 (b, 18H, TIPS); 1.22 (s, 3H, H-19); 3.37 (dd, J=10 Hz, 7 Hz, 1H, H-22); 3.65 (dd, J=10 Hz, 3 Hz, 1H, H-22); 4.18 (b, 1 H, H-3)

d) (20S)-20-(triisopropylsilyloxymethyl)-5β-pregnan-5,3β-carbolactone

7.12 g (20S)-5-cyano-20-(triisopropylsilyloxymethyl)-5β-pregnan-3β-ol were reduced with diisobutylaluminumhydride as decribed in example 1a. After work up and chromatography 2.59 g (20S)-20-(triisopropylsilyloxymethyl)-5β-pregnan-5,3β-carbolactol were isolated. This material is oxidized with chromiumtrioxide pyridine complex as described in example 1b to give 2.21 g (20S)-20-(triisopropylsilyloxymethyl)-5β-pregnan-5,3β-carbolactone after column chromatography.

$^1$H-NMR ($CDCl_3$): δ = 0.68 (s, 3H, H-18); 1.00 (s, 3H, H-19); 1.02 (d, J=7 Hz, 3H, H-21); 1.05 (b, 18H, TIPS); 2.40 (d, J = 11 Hz, 1H, H-4); 3.37 (dd, J=10 Hz, 7 Hz, 1H, H-22); 3.65 (dd, J=10 Hz, 3 Hz, 1H, H-22); 4.71 (b, 1 H, H-3)

**e) (20S)-20-(hydroxymethyl)-5β-pregnan-5,3β-carbolactone**

A solution of 2.10 g (20S)-20-(triisopropylsilyloxymethyl)-5β-pregnan-5,3β-carbolactone and 1.92 g tetrabutylammonium fluoride trihydrate in 30 ml tetrahydrofuran is stirred for three hours at room temperature. The mixture is diluted with ethyl acetate, washed with water and brine, dried over sodium sulfate, filtered and evaporated in vacuo. After column chromatography 1.45 g (20S)-20-(hydroxymethyl)-5β-pregnan-5,3β-carbolactone were isolated.

$^1$H-NMR ($CDCl_3$): δ = 0.70 (s, 3H, H-18); 1.00 (s, 3H, H-19); 1.04 (d, J=7 Hz, 3H, H-21); 2.40 (d, J= 11 Hz, 1H, H-4); 3.37 (m, 1H, H-22); 3.65 (m, 1H, H-22); 4.71 (b, 1 H, H-3)

f) (20S)-20-(p-toluenesulfonyloxymethyl)-5β-pregnan-5,3β-carbolactone

1.12 g (20S)-20-(hydroxymethyl)-5β-pregnan-5,3β-carbolactone were treated with p-toluenesulfonic chloride as described in example 7d. After work up and chromatography 1.30 g (20S)-20-(p-toluenesulfonyloxymethyl)-5β-pregnan-5,3β-carbolactone were isolated as a white solid.

$^1$H-NMR ($CDCl_3$): δ = 0.63 (s, 3H, H-18); 0.96 (s, 3H, H-19); 0.98 (d, J=7 Hz, 3H, H-21); 2.40 (d, J = 11 Hz, 1H, H-4); 2.45 (s, 3H, aryl-$CH_3$); 3.72 (dd, J= 10 Hz, 5 Hz, 1H, H-22); 3.94 (dd, J= 10 Hz, 3 Hz, 1H, H-22); 4.70 (b, 1 H, H-3); 7.35 (d, J= 8 Hz, 2H, aryl); 7.77 (d, J= 8 Hz, 2H, aryl)

g) 24-nor-5β-cholestane-5,3β-carbolactone

80 mg (20S)-20-(p-toluenesulfonyloxymethyl)-5β-pregnan-5,3β-carbolactone were treated with isobutylmagnesium bromide in the presence of a copper catalyst as described in example 7e. After work up and chromatography 19 mg 24-nor-5β-cholestane-5,3β-carbolactone were isolated as a white solid.

$^1$H-NMR ($CDCl_3$): δ = 0.68 (s, 3H, H-18); 0.85-0.92 (3xd, 9H, H-21/25/26); 0.98 (s, 3H, H-19); 2.40 (d, J = 11 Hz, 1 H, H-4); 4.70 (b, 1 H, H-3)

**Example 11: (20R)-21-cyclohexyl-20-methyl-5β-pregnane-5,3β-carbolactone**

144 mg (20S)-20-(p-toluenesulfonyloxymethyl)-5β-pregnan-5,3β-carbolactone were treated with cyclohexylmagnesium bromide in the presence of a copper catalyst analogously to example 7e. After work up and chromatography 82 mg (20R)-21-cyclohexyl-20-methyl-5β-pregnane-5,3β-carbolactone were isolated as a white solid.

$^1$H-NMR ($CDCl_3$): δ = 0.68 (s, 3H, H-18); 0.88 (d, J= 7 Hz, 3H, H-21); 0.99 (s, 3H, H-19); 2.40 (d, J= 12 Hz, 1H, H-4); 4.70 (b, 1 H, H-3)

**Example 12: 17β-(prenyloxy)-5β-androstane-5,3β-carbolactone**

a) 17β-(triisopropylsilyloxy)androst-4-en-3-one

10.09 ml triisopropylsilylchloride were added to a solution of 10.0 g testosterone and 5.16 g imidazol in 115 ml dichloromethane. The reaction mixture was stirred for 3 days at room temperature. Work out and purification were carried out as described in example 10a. 8.6 g 17β-(triisopropylsilyloxy)androst-4-en-3-one were isolated as a white solid.

$^1$H-NMR ($CDCl_3$): δ = 0.78 (s, 3H, H-18); 1.02 (b, 12H, TIPS); 1.18 (s, 3H, H-19); 3.72 (t, J= 7 Hz, 1H, H-3), 5.72 (s, 1H, H-4)

b) 5-cyano-17β-(triisopropylsilyloxy)-5β-androstan-3-one

8.6 g 17β-(triisopropylsilyloxy)androst-4-en-3-one were treated with diethylaluminum cyanide as was described in example 7c. After work up and chromatography 3.04 g 5-cyano-17β-(triisopropylsilyloxy)-5β-androstan-3-one were isolated as a white solid.

$^1$H-NMR ($CDCl_3$): δ = 0.78 (s, 3H, H-18); 1.02 (b, 12H, TIPS); 1.28 (s, 3H, H-19); 2.99 (d, J = 16 Hz, 1H, H-4); 3.73 (t, J = 7 Hz, 1H, H-3)

c) 5-cyano-17β-(triisopropylsilyloxy)-5β-androstan-3β-ol

3.03 g 5-cyano-17β-(triisopropylsilyloxy)-5β-androstan-3-one were reduced with 13 ml K-selectride solution as was described in example 10c. After work up and column chromatography 2.69 g 5-cyano-17β-(triisopropylsilyloxy)-5β-androstan-3β-ol were isolated as a white solid.

$^1$H-NMR ($CDCl_3$): δ = 0.73 (s, 3H, H-18); 1.03 (b, 12H, TIPS); 1.22 (s, 3H, H-19); 3.71 (t, J= 7 Hz, 1H, H-3); 4.19 (m, 1H, H-3)

d) **17β-(triisopropylsilyloxy)-5β-androstane-5,3β-carbolactone**

2.69 g 5-cyano-17β-(triisopropylsilyloxy)-5β-androstan-3β-ol were reduced with diisobutylaluminum hydride solution as was decribed in example 10d to give 1.4 g of 17β-(triisopropylsilyloxy)-5β-androstane-5,3β-carbolactol, which was oxidized immediately with chromiumtrioxide pyridine complex to give 1.4 g 17β-(triisopropylsilyloxy)-5β-androstane-5,3β-carbolactone after work up and column chromatography.

$^1$H-NMR ($CDCl_3$): δ = 0.75 (s, 3H, H-18); 1.00 (s, 3H, H-19);1.05 (b, 12H, TIPS); 2.38 (d, J= 13 Hz, 1H, H-4); 3.74 (t, J= 7 Hz, 1H, H-3); 4.71 (t, J= 5 Hz, 1H, H-3)

e) 17β-hydroxy-5β-androstane-5,3β-carbolactone

1.4 g 17β-(triisopropylsilyloxy)-5β-androstane-5,3β-carbolactone wer desilylated with tetrabutylammonium fluoride as was described in example 10e. After work up and column chromatography 676 mg 17β-hydroxy-5β-androstane-5,3β-carbolactone were isolated as a white solid.

$^1$H-NMR ($CDCl_3$): δ = 0.75 (s, 3H, H-18); 1.01 (s, 3H, H-19); 2.38 (d, J = 13 Hz, 1H, H-4); 3.65 (t, J= 8 Hz, 1H, H-3); 4.71 (t, J= 5 Hz, 1H, H-3)

f) 17β-(prenyloxy)-5β-androstane-5,3β-carbolactone

30 mg sodium hydride [60 % in mineral oil] were added to a solution of 100 mg 17β-hydroxy-5β-androstane-5,3β-carbolactone in 3 ml dimethylformamide at room temperature. Then 0.11 ml prenylbromide were added dropwise. The reaction mixture was stirred for 20 hours. Water and ethyl acetate were added, the organic extracts were washed with brine, dried over anhydrous sodium sulfate and evaporated in vacuo. The residue was chromatographed to give 20 mg 17β-(prenyloxy)-5β-androstane-5,3β-carbolactone as a white solid.

$^1$H-NMR ($CDCl_3$): δ = 0.78 (s, 3H, H-18); 1.00 (s, 3H, H-19); 2.39 (d, J= 12 Hz, 1H, H-4); 3.36 (t, J= 8 Hz, 1H, H-3); 3.98 (m, 2H, $OCH_2$); 4.71 (t, J= 5 Hz, 1H, H-3); 5.34 (m, 1 H, olefin)

**Example 13: Testing of meiosis-activating substances in the oocyte test**

Animals

Oocytes were obtained from immature female mice (C57Bl/6J × DBA/2J F1-hybrids, Bomholtgaard, Denmark) weighing 13 - 16 grams, that were kept under controlled lighting and temperature. The mice received an intraperitoneal injection of 0.2 ml gonadotropins (Gonal F, Serono, Solna, Sweden , containing 20 IU FSH, alternatively, Puregon, Organon, Swords, Ireland containing 20 IU FSH) and 48 hours later the animals were killed by cervical dislocation.

Collection and culture of oocytes

The ovaries were dissected out and the oocytes were isolated in Hx-medium (see below) under a stereo microscope by manual rupture of the follicles using a pair of 27 gauge needles. Spherical, naked oocytes (NO) displaying an intact germinal vesicle (GV) were placed in α-minimum essential medium (α-MEM without ribonucleosides, Gibco BRL, Cat.No. 22561) supplemented with 3 mM hypoxanthine (Sigma Cat. No. H-9377), 8 mg/ml Human Serum Albumin (HSA, State Serum Institute, Denmark), 0,23 mM pyrubate (Sigma, Cat. No. S-8636), 2 mM glutamine (Flow Cat. No. 16-801), 100 IU/ml penicillin and 100 μg/ml streptomycin (Flow, Cat No. 16-700). This medium was designated Hx-medium.

The oocytes were rinsed three times in Hx-medium and cultured in 4-well multidishes (Nuncion, Denmark) in which each well contained 0.4 ml of Hx-medium and 30 - 40 oocytes. One control (i.e. 30 - 40 oocytes cultured in Hx-medium

with no addition of test compound) was always run simultaneously with the test cultures, which were made with different concentrations of the compounds to be tested.
The cultures were performed at 37 °C and 100 % humidity with 5 % $CO_2$ in air. The culture time was 22 - 24 hours.

**Examination of oocytes**

By the end of the culture period, the number of oocytes with germinal vesicle (GV) or germinal vesicle breakdown (GVB) and those with polar body (PB) was counted using a stereo microscope or an inverted microscope with differential interference contrast equipment. The percentage of oocytes with GVB per total number of oocytes and the percentage of oocytes with PB per total number of oocytes was calculated in the test cultures and compared to the control culture.

**Example 14: Test of meiosis-inhibiting substances in the oocyte test**

Germinal vesicle (GV) oocytes were obtained from immature FSH treated female mice using the same methods as described in Example 1 (see above). Naked oocytes (NO) were rinsed three times in Hx-medium. 4,4-Dimethylcholest-B, 14,24-trien-3β-ol (FF-MAS) has previously been shown to induce meiosis in NO invitro (Byskov, A.G. et al. Nature **374** (1995) 559 - 562). NO were cultured in Hx-medium supplemented with 5 μM FF-MAS in co-culture with the test compounds in different concentrations in 4-well multidishes (Nunclon, Denmark) in which each well contained 0.4 ml of Hx-medium and 30 - 40 oocytes. One positive control (i.e., 30 - 40 oocytes cultured in Hx-medium containing FF-MAS with no addition of test compound) was always run simultaneously with the test cultures, which were supplemented with different concentrations of the compounds to be tested. In addition, one negative control ( 30 - 40 oocytes cultured in Hx-medium alone) was run simultaneously with the positive control.

Examination of oocytes

By the end of the culture period, the number of oocytes with germinal vesicle (GV) or germinal vesicle breakdown (GVB) and those with polar body (PB) was counted using a stereo microscope or an inverted microscope with differential interference contrast equipment. The percentage of oocytes with GVB + PB per total number of oocytes were calculated in the test cultures and in the control (positive and negative) culture groups. The relative inhibition of the test compound was calculated by the following formula:

inhibition test compound (%) = 100% - [(GVB+PB (test compound) -

GVB+PB (negative control)) x 100 / GVB+PB(positive control) - GVB+PB (negative control)] %

Oocytes arrested in meiosis are characterised by an intact nucleus with a prominent nucleolus, known as germinal vesicle (GV). Upon reinitiation of meiosis the nucleolus and the nuclear envelope disappear and this is characterised by a breakdown of the GV, which than is called germinal vesicle breakdown (GVB). Some hours later the oocyte complete a reductional division and elicit the first so called polar body (PB).

Results:

Table 1: Relative inhibition [%] of meiosis in naked mouse oocytes

| Compounds | Oocytes [n] | | | Inhibition [%] |
|---|---|---|---|---|
| | GV | GVB | PB | |
| Control (Hx) | 31 | 0 | 7 | 100 |
| 5µM FF-MAS | 4 | 16 | 16 | 0 |
| 5µM FF-MAS + 10 µM 17β-(prenyloxy)-5β-androstane-5,3β-carbolactone (example 12) | 24 | 9 | 1 | 84 |
| Control (Hx) | 40 | 0 | 0 | 100 |
| 5µM FF-MAS | 8 | 25 | 6 | 0 |
| 5µM FF-MAS + 10 µM 2-[(4aα-methyl-4a-homo-3α,5-methano-4-oxa-5α-cholest-8-en-3β-yl)oxy]ethanol (example 5) | 30 | 0 | 0 | 100 |

Hx = Hypoxanthine

GV = germinal vesicle

GVB = germinal vesicle breakdown

PB = polar bodies

n = number of oocytes

Table 2: Activation of meiosis in naked mouse oocytes

| Compounds | Oocytes [n] | | | Activation [%] |
|---|---|---|---|---|
| | GV | GVB | PB | GVB+PB |
| Control (Hx) | 40 | 0 | 0 | 0 |
| 10 μM FF-MAS | 6 | 31 | 2 | 85 |
| 1 μM ZK 255276 | 21 | 10 | 4 | 40 |
| 3 μM ZK 255276 | 19 | 11 | 5 | 46 |
| 10 μM ZK 255276 | 0 | 30 | 0 | 100 |

Hx = Hypoxanthine

GV = germinal vesicle

GVB = germinal vesicle breakdown

**Claims**

1. Compounds of the general formula I

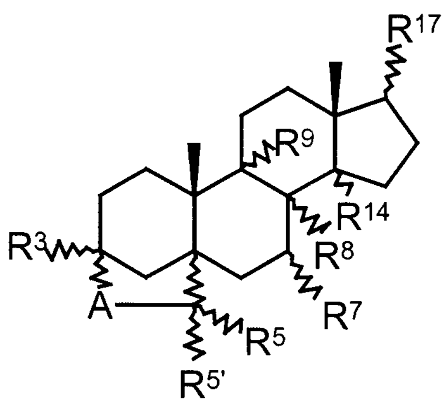

I

wherein

$R^3$ designates a hydrogen atom, a hydroxy group or a 2-hydroxyethoxy group,
A designates an oxygen atom or a NH group,
$R^5$ designates a hydrogen atom, a hydroxy group, an $C_1$-$C_8$ linear or branched alkyl or alkenyl group, a $C_6$-$C_{10}$ aryl group or together with $R^{5'}$ an oxo group,
**$R^{5'}$** designates a hydrogen atom or together with $R^5$ an oxo group,
$R^7$ designates a hydrogen atom or together with $R^8$ an additional bond,
$R^8$ designates a hydrogen atom or together with R7 or together with R9 or together with $R^{14}$ an additional bond,
$R^9$ designates a hydrogen atom or together with $R^8$ an additional bond,
$R^{14}$ designates a hydrogen atom or together with $R^8$ or together with $R^{15}$ an additional bond,
$R^{17}$ a $C_1$-$C_{10}$ linear or branched optionally substituted alkyl or alkenyl group, an optionally substituted $C_8$-$C_{12}$

arylalkyl group, a $C_1$-$C_{10}$ linear or branched optionally substituted alkoxy group or an optionally substituted $C_6$-$C_{10}$ arylalkoxy group

or isomeric forms, salts or esters thereof. with the provisio that said compounds are not 5β-cholestane-5, 3β-carbolactone, 5α-cholestane-5,3α-carbolactone, 3α-hydroxy-5β-cholestane-5,3β-carbolactame, 3β-hydroxy-5α-cholestane-5, 3α-carbolactame or 3α-(2-hydroxyethoxy)-5β-cholestane-5,3β-carbolactame.

**2.** Compounds according to claim 1, wherein $R^3$ designates a hydrogen atom or a hydroxy group

**3.** Compounds according to claim 1 or 2, wherein $R^5$ and $R^{5'}$ designate together an oxo group.

**4.** Compounds according to any one of the claims 1 to 3, wherein $R^{17}$ designate a a $C_1$-$C_{10}$ linear or branched optionally substituted alkyl or alkenyl group or a $C_1$-$C_{10}$ linear or branched optionally substituted alkoxy group.

**5.** Compounds to any one of the claims 1 to 4 which are

5β-cholest-8-ene-5,3β-carbolactone
5β-cholest-8(14)-ene-5,3β-carbolactone
3α-hydroxy-5β-cholest-8-ene-5,3β-carbolactame
3α-hydroxy-5β-cholest-8(14)-ene-5,3β-carbolactame
2-[(4aα-methyl-4a-homo-3α,5-methano-4-oxa-5α-cholest-8-en-3β-yl)oxy]ethanol
2-[(4aα-ethyl-4a-homo-3α,5-methano-4-oxa-5α-cholest-8-en-3β-yl)oxy]ethanol
2-[(4aα-methyl-4a-homo-3α,5-methano-4-oxa-24-nor-5α-cholest-8-en-3β-yl)oxy]ethanol
2-[(20R)-(4aα,20-dimethyl-4a-homo-3α,5-methano-4-oxa-21-phenyl-5α-pregn-8-en-3β-yl)oxy]ethanol
2-[(20R)-(21-cyclohexyl-4aα,20-dimethyl-4a-homo-3α,5-methano-4-oxa-5α-pregn-8-en-3β-yl)oxy]ethanol
2-[(4aα-methyl-4a-homo-3α,5-methano-4-oxa-5α-cholest-8(14)-en-3β-yl)oxy]ethanol
24-nor-5β-cholestane-5,3β-carbolactone
(20R)-21-cyclohexyl-20-methyl-5β-pregnane-5,3β-carbolactone
17β-(prenyloxy)-5β-androstane-5,3β-carbolactone

**6.** Pharmaceutical compositions comprising one or more compounds of the general formula I according to any one of the claims 1 to 5 together with a pharmaceutically acceptable excipient.

**7.** Use of the compounds of the general formula II

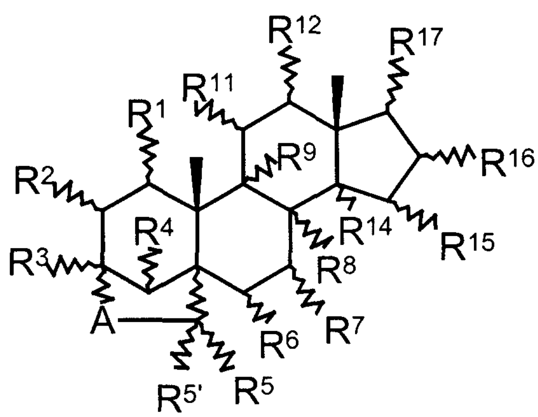

II

wherein

| | |
|---|---|
| $R^3$ | designates a hydrogen atom, a hydroxy group or a 2-hydroxyethoxy group, |
| A | designates an oxygen atom or a NH group, |

| | |
|---|---|
| $R^5$ | designates a hydrogen atom, a hydroxy group, an $C_1$-$C_8$ linear or branched alkyl or alkenyl group, a $C_6$-$C_{10}$ aryl group or together with $R^{5'}$ an oxo group, |
| $R^{5'}$ | designates a hydrogen atom or together with $R^5$ an oxo group, and |
| R1, R2, R4, R6-R9, R11, R12 and R14-R17 | designate any substituent, for the preparation of a meiosis-regulating medicament. |

**8.** Use according to claim 7 wherein

| | |
|---|---|
| $R^3$ | designates a hydrogen atom, a hydroxy group or a 2-hydroxyethoxy group, |
| A | designates an oxygen atom or a NH group, |
| $R^5$ | designates a hydrogen atom, a hydroxy group, an $C_1$-$C_8$ linear or branched alkyl or alkenyl group, a $C_6$-$C_{10}$ aryl group or together with $R^{5'}$ an oxo group, |
| $R^{5'}$ | designates a hydrogen atom or together with $R^5$ an oxo group, |
| $R^7$ | designates a hydrogen atom or together with $R^8$ an additional bond, |
| $R^8$ | designates a hydrogen atom or together with $R^7$ or together with $R^9$ or together with $R^{14}$ an additional bond, |
| $R^9$ | designates a hydrogen atom or together with $R^8$ an additional bond, |
| $R^{14}$ | designates a hydrogen atom or together with R8 or together with $R^{15}$ an additional bond, |
| $R^{17}$ | a $C_1$-$C_{10}$ linear or branched optionally substituted alkyl or alkenyl group, an optionally substituted $C_8$-$C_{12}$ arylalkyl group, a $C_1$-$C_{10}$ linear or branched optionally substituted alkoxy group or an optionally substituted $C_6$-$C_{10}$ arylalkoxy group and |
| R1, R2, R4, R6, R11, R12, R15 and R16 | designate a hydrogen atom in the general formula II. |

**9.** Use according to claim 7 or 8 wherein $R^3$ designates a hydrogen atom or a hydroxy group in the general formula II.

**10.** Use according to any one of the claims 7-9 wherein $R^5$ and $R^{5'}$ designate together an oxo group in the general formula II.

**11.** Use according to any one of the claims 7-10 wherein $R^{17}$ in the general formula II designate a $C_1$-$C_{10}$ linear or branched optionally substituted alkyl or alkenyl group or a $C_1$-$C_{10}$ linear or branched optionally substituted alkoxy group.

**12.** Use according to any one of the claims 7-11 for the preparation of a contraceptive medicament for the treatment of females or males, preferably humans.

**13.** A method of regulating meiosis comprising administering to a subject in need of such a regulation an effective amount of one or more compounds of the general formula II according to any one of claims 7-11.

European Patent Office

**PARTIAL EUROPEAN SEARCH REPORT**
which under Rule 45 of the European Patent Convention shall be considered, for the purposes of subsequent proceedings, as the European search report

Application Number
EP 00 25 0059

**DOCUMENTS CONSIDERED TO BE RELEVANT**

| Category | Citation of document with indication, where appropriate, of relevant passages | Relevant to claim | CLASSIFICATION OF THE APPLICATION (Int.Cl.7) |
|---|---|---|---|
| X | W. NAGATA ET AL: "Angular Substituted Polycyclic Compounds. I. Cyanation of delta-4-Cholesten-3-one" JOURNAL OF ORGANIC CHEMISTRY., vol. 26, no. 7, 25 July 1961 (1961-07-25), pages 2413-2420, XP002142047 AMERICAN CHEMICAL SOCIETY. EASTON., US ISSN: 0022-3263 * page 2415, compound VIIIc * --- | 1,3 | C07J71/00 A61K31/58 A61P15/08 A61K31/585 |
| X | NAGATA, WATARU ET AL: "Synthesis of bridged steroids. III. Cholestane derivatives having a bridged bicyclo‘2.2.2!octane ring system of the atisine type" CHEM. PHARM. BULL. (TOKYO) (1968), 16(5), 885-96 , XP002142048 * the whole document * --- | 1,4 | |
| | -/-- | | TECHNICAL FIELDS SEARCHED (Int.Cl.7) |
| | | | C07J A61K A61P |

**INCOMPLETE SEARCH**

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claim 13 is directed to a method of treatment of the human/animal body (Article 52(4) EPC), the search has been carried out and based on the alleged effects of the compound/composition.

| Place of search | Date of completion of the search | Examiner |
|---|---|---|
| THE HAGUE | 28 July 2000 | Watchorn, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 00 25 0059

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | CHEMICAL ABSTRACTS, vol. 57, no. 13, 24 December 1962 (1962-12-24) Columbus, Ohio, US; abstract no. 16691f, W. NAGATA ET AL: "Alkaline degradation of 3.alpha.-alkoxy-3.beta.-amino-5.beta.-cholestan-5-carboxylic acid gamma-lactam" XP002142051 * abstract * & CHEMICAL AND PHARMACEUTICAL BULLETIN, vol. 9, 1961, pages 845-854, TOKYO JP --- | 1,2,4 | |
| | | | TECHNICAL FIELDS SEARCHED (Int.Cl.7) |
| X | CHEMICAL ABSTRACTS, vol. 70, no. 5, 3 February 1969 (1969-02-03) Columbus, Ohio, US; abstract no. 20275, NAGATA, WATARU ET AL: "Synthesis of bridged steroids. IV. Cholestane derivatives with a 3,5-(1-azaethano) bridge" XP002142052 * abstract * * in particular compounds X and XI * & CHEM. PHARM. BULL. (TOKYO) (1968), 16(8), 1550-5 , --- | 1-4 | |
| X | FAJKOS, JAN ET AL: "Steroids. CCXCI. The bromination of the epimeric 4,5-cyclopropanocholestan-3-ones" COLLECT. CZECH. CHEM. COMMUN. (1983), 48(12), 3455-73 , XP002142049 * page 3458, compound XXXVII ; page 3460, compound XL * --- | 1,2,4 | |

-/--

EPO FORM 1503 03.82 (P04C10)

European Patent Office

# PARTIAL EUROPEAN SEARCH REPORT

Application Number
EP 00 25 0059

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| X | ARENCIBIA, MARIA T. ET AL: "Hypervalent organoiodine compounds: radical fragmentation of oxabicyclic hemiacetals. Convenient synthesis of medium-sized and spiro lactones" J. CHEM. SOC., PERKIN TRANS. 1 (1991), (12), 3349-60 , XP002142050 * page 3352, compounds 49 and 51 * --- | 1,2,4 | |
| A | EP 0 957 108 A (SCHERING AG) 17 November 1999 (1999-11-17) * page 4, line 29 - line 34 * * page 2, paragraph 6 - paragraph 8 * --- | 1-13 | TECHNICAL FIELDS SEARCHED (Int.Cl.7) |
| A | US 3 478 150 A (NARISADA MASAYUKI) 11 November 1969 (1969-11-11) * column 2, paragraph 2; examples V,VI,VIII * --- | 1-13 | |
| A | US 3 120 515 A (R. G. CHRISTIANSEN) 4 February 1964 (1964-02-04) * column 2, formula IV, column 3, formulae VI and IX * * column 4, paragraph 2; examples 7-11,19,20,25,26 * --- | 1-13 | |
| A | US 3 200 113 A (R. G. CHRISTIANSEN) 10 August 1965 (1965-08-10) * column 4, formula XIV * * page 5, paragraph 2; examples 9,10 * ----- | 1-13 | |

EPO FORM 1503 03.82 (P04C10)

**ANNEX TO THE EUROPEAN SEARCH REPORT ON EUROPEAN PATENT APPLICATION NO.** EP 00 25 0059

This annex lists the patent family members relating to the patent documents cited in the above-mentioned European search report.
The members are as contained in the European Patent Office EDP file on
The European Patent Office is in no way liable for these particulars which are merely given for the purpose of information.

28-07-2000

| Patent document cited in search report | Publication date | Patent family member(s) | Publication date |
|---|---|---|---|
| EP 0957108 A | 17-11-1999 | AU 3701199 A | 29-11-1999 |
| | | WO 9958549 A | 18-11-1999 |
| US 3478150 A | 11-11-1969 | CH 478783 A | 30-09-1969 |
| | | DE 1493076 A | 07-08-1969 |
| | | FR 6159 M | 08-07-1968 |
| | | FR 1440553 A | 31-08-1966 |
| | | GB 1052478 A | |
| US 3120515 A | 04-02-1964 | NONE | |
| US 3200113 A | 10-08-1965 | NONE | |

EPO FORM P0459

For more details about this annex : see Official Journal of the European Patent Office, No. 12/82